# EUROPÄISCHE PATENTANMELDUNG

(11) **EP 0 534 198 A2**
(43) Veröffentlichungstag der Anmeldung: **31.03.1993**
(21) Anmeldenummer: 92115145.2
(22) Anmeldetag: 04.09.1992
(51) Int. Cl.: H04N 5/232, H04N 5/225

(54) **Endoskop mit einer Festkörper-Bildaufnahmevorrichtung versehenen auswechselbaren Kameraköpfen**

(30) Priorität: 27.09.1991 DE 4132125
(71) Anmelder: Richard Wolf GmbH, D-75438 Knittlingen (DE)
(72) Erfinder: Häfele, Ulrich, W-7137 Sternenfels-Diefenbach (DE); Vögele, Michael, W-7539 Kämpfelbach-Ersingen (DE)
(74) Vertreter: Vollmann, Heiko, Dipl.-Ing.

(57) **Zusammenfassung**

Das Endoskop weist einen elektronischen Bildwandler (4) auf. Es ist mit einem Steuergerät (1) verbindbar, an dem ein Monitor zur Bildwiedergabe anschließbar ist. Dem Bildwandler (4) ist endoskopseitig ein statischer Speicher (3) zugeordnet, der als Schreib- und Lesespeicher ausgebildet ist und der zur Speicherung aller für das Steuergerät (1) erforderlichen Abgleichparameter des Bildwandlers (4) vorgesehen ist. Der Speicher (3) ist mittels des Steuergerätes (1) belegbar und wieder abfragbar.

Auch die Betriebsparameter und/oder weitere Daten, wie beispielsweise herstellerspezifische oder Service-Daten sind im Speicher ablegbar. Eine Weiterbildung ermöglicht es sogar individuelle Betriebsparameter, wie Farbe, Helligkeit, Kontrast endoskopseitig zu speichern, so daß die vom Anwender gewählte Einstellungsparameter beim erneuten Einsatz des Instruments, beispielsweise nach der Desinfektion, wieder zur Verfügung stehen, wodurch gerade der Einsatz unterschiedlicher Endoskope mit unterschiedlichen Bildwandlern und Betriebsparametern deutlich vereinfacht wird.

## Beschreibung

Die Erfindung geht aus von einem Endoskop mit elektronischem Bildwandler, das über ein damit verbindbares Steuergerät an einen Monitor zur Bildwiedergabe anschließbar ist, mit einem dem Bildwandler endoskopseitig zugeordneten statischen Speicher.

Bei derartigen bekannten Endoskopen ist entweder proximalseitig eine Videokamera aufgesetzt oder aber, bei sogenannten Videoendoskopen, ein elektronischer Bildwandler, z.B. ein CCD im distalen Endbereich des Instrumentes angeordnet. Die vom Bildwandler (der Videokamera oder des Endoskopes selbst) abgegebenen elektrischen Signale werden in einem Kamera- bzw. Videosteuergerät verarbeitet, um dann beispielsweise einen Farbmonitor zur entsprechenden Bildwiedergabe anzusteuern. Da an einem solchen Kamera- bzw. Videosteuergerät verschiednene Kameraköpfe bzw. Videoendoskope betrieben werden sollen, müssen die Bildwandler der unterschiedlichen Kameraköpfe bzw. Videoendoskope, die sich in ihren elektrischen Anschlußwerten erheblich unterscheiden können, an das Steuergerät angepaßt werden, um eine optimale Bildübertragung sicherzustellen.

Die Anpassung am Kamera- bzw. Videoendoskop wird üblicherweise mit Hilfe relativ aufwendiger elektronischer Schaltungen vorgenommen, die im Endoskop oder in der Kamera untergebracht werden müssen. Zum Einstellen oder Verändern dieser wandlerspezifischen Anpaßwerte ist eine zumindest teilweise Demontage der Bildaufnahmevorrichtung und des Steuergerätes nötig, um die entsprechenden Einstellungen und Messungen vornehmen zu können. Dies schließt in der Regel eine Veränderung der vorgegebenen Werte bzw. die Anpassung eines Videoendoskops an ein vorgegebenes Steuergerät durch den Anwender aus.

Aus DE 37 42 900 C2 ist eine Videoendoskopvorrichtung bekannt, an deren Steuergerät Videoendoskope mit Bildwandlern unterschiedlicher Bildpunktzahl betrieben werden können. Die Anpaßschaltung ist dort weitgehend im Steuergerät integriert, wobei eine Anpassung auch nur hinsichtlich der Bildpunktzahl des eingesetzten Bildwandlers erfolgt. Diese Bildpunktzahl ist in einem endoskopseitigen Lesespeicher abgelegt, der innerhalb des Steckers angeordnet ist, mit dem das Videoendoskop mit dem zugehörigen Steuergerät verbunden ist. Bei diesem Stand der Technik kann von einer vollständigen Anpassung des Bildwandlers an das Steuergerät bzw. umgekehrt keine Rede sein, denn eine Anpassung erfolgt nur im Hinblick auf die unterschiedliche Bildpunktzahl des Bildwandlers. Die hier zu verwendenden Bildwandler sind fest vorgegeben, so daß das Steuergerät nach der Abfrage der Bildpunktzahl alle weiteren Werte nach vorgegebenen Einstellungen wählt. Da bei dieser Vorrichtung keine vollständige Anpassung zwischen Bildwandler und Steuergerät erfolgt, kann dieses Steuergerät nur mit einer fest vorgegebenen, in der Regel kleinen Anzahl von Bildwandlern, sei es in Videoendoskopen oder in Kameras betrieben werden. Zudem ist eine Selektierung der verwendeten Bildwandler erforderlich, da eine Anpassung des Steuergeräts zwar an den jeweils angeschlossenen Bildwandlertyp, nicht jedoch individuell zwischen gleichen Typen differenzierend erfolgt. Auch ist es nicht möglich, mit derartigen Videoendoskopvorrichtungen subjektiv gewählte Einstellparameter, wie beispielsweise Helligkeit, Farbe oder Kontrast des endoskopischen Bildes einem bestimmten Bildwandler und somit einer bestimmten Kamera oder einem bestimmten Videoendoskop zuzuordnen, um beispielsweise nach der Desinfektion des Endoskops beim nächsten Eingriff auf diese einmal gewählten Einstellungen wieder zurückgreifen zu können.

Ausgehend von dem vorgenannten Stand der Technik liegt der Erfindung die Aufgabe zugrunde, ein gattungsgemäßes Endoskop so weiter zu bilden, daß zum einen ein vom eingesetzten Bildwandler praktisch unabhängiger Betrieb mit einem entsprechenden Steuergerät möglich ist und gegebenenfalls die Abgleichparameter auch später noch durch das Steuergerät verändert werden können. In einer Weiterbildung der Erfindung sollen zudem individuelle Einstellparameter auf möglichst einfache Weise reproduzierbar sein.

Gemäß der Erfindung wird dies dadurch erreicht, daß als Speicher ein statischer Schreib- und Lesespeicher eingesetzt wird, der sämtliche für das Steuergerät erforderlichen Abgleichparameter des Bildwandlers aufnimmt und der mittels des Steuergerätes nicht nur lesbar, sondern auch belegbar und gegebenenfalls wiederbelegbar ist.

Die erfindungsgemäße Lösung hat daher nicht nur den besonderen Vorteil, daß praktisch jeder Bildwandler in die Endoskopkamera oder das Videoendoskop eingesetzt werden kann, ohne daß eine Anpassung des Steuergeräts erforderlich ist, sondern sie ermöglicht zudem auch den Einsatz von zukünftigen Bildwandlern, deren elektrische Anschlußdaten derzeit noch nicht feststehen. Die Erfindung ermöglicht weiterhin, beispielsweise nach längerer Betriebszeit, einen erneuten Abgleich durchzuführen und diese Daten innerhalb des Speichers abzulegen. All das kann mittels des Steuergerätes erfolgen, ohne daß irgendwelche baulichen Veränderungen endoskop- oder steuergeräteseitig erforderlich sind. Ein solcher Abgleich kann daher schnell und einfach vom Anwender durchgeführt werden.

Eine bevorzugte Weiterbildung der Erfindung sieht vor, daß nicht nur die Abgleichparameter im Speicher ablegbar sind, sondern auch die Betriebsparameter und/oder weitere Daten, wie beispielsweise herstellerspezifische oder Service-Daten. Diese Weiterbildung ermöglicht es sogar individuelle Betriebsparameter, wie Farbe, Helligkeit, Kontrast usw. endoskopseitig zu speichern, so daß die vom Anwender gewählten Einstellungsparameter beim erneuten Einsatz des Instrumentes, beispielsweise nach der Desinfektion, wieder zur Verfügung stehen, wodurch gerade der Einsatz unterschiedlicher Endoskope mit unterschiedlichen Bildwandlern und Betriebsparametern deutlich vereinfacht wird.

Als statischer Schreib- und Lesespeicher wird bevorzugt ein ECONORAM und zwar der Typ DS222X von der Herstellerfirma DALLAS SEMICONDUCTOR, USA eingesetzt. Diese Bauart hat nur drei Anschlüsse (Signalein- und -ausgang, Versorgungsspannung und Masse), so daß in der Praxis nur eine zusätzliche (Signal-) Leitung - Spannungsversorgung und Masseanschluß kann über die entsprechenden Anschlüsse des CCD-Sensors erfolgen - neben den ohnehin für den Anschluß des CCD-Sensors erforderlichen Leitungen benötigt wird. Zum Lesen und Belegen des Speichers ist nur eine weitere Leitung erforderlich. Da das vorerwähnte Bauteil nur geringe Abmessungen aufweist, kann es zudem im Stecker oder an beliebiger endoskopseitiger Stelle angeordnet sein.

Es versteht sich, daß das erfindungsgemäße Endoskop, soweit eine Bildwandleranpassung zum Steuergerät gegeben ist, auch mit üblichen Steuergeräten eingesetzt werden kann. Zweckmäßigerweise sollte das Steuergerät jedoch zur Abfrage des endoskopseitigen Speichers ausgebildet sein, so daß steuergeräteseitig nach Abfrage der Speicherdaten eine Abgleichung an den Bildwandler erfolgen kann. Bevorzugt ist das Steuergerät so ausgebildet, daß es nicht nur die im endoskopseitigen Speicher abgelegten Daten lesen und die steuergeräteseitigen Einstellungen entsprechend vornehmen kann, sondern daß diese Parameter zumindest teilweise auch steuergeräteseitig vorgegeben und so in den endoskopseitigen Speicher schreibbar sind. Insbesondere für die Speicherung der Betriebsdaten ist dies zweckmäßig.

So kann es herstellerseitig vorgesehen sein, daß die im Handel befindlichen Steuergeräte nur zur Abspeicherung der Abgleich- und Betriebsparameter im endoskopseitigen Speicher geeignet sind und lediglich die Werkstattgeräte auch zur Speicherung der weiteren Daten benutzt werden können.

Als weitere Daten, die im endoskopseitigen Speicher ablegbar sind, kommen beispielsweise Daten über die Betriebsdauer, die Wartungsintervalle, die Seriennummer und dergleichen in Frage.

Das erfindungsgemäße Endoskop mit Steuergerät wird nachstehend anhand eines in der Zeichnung dargestellten Ausführungsbeispiels näher erläutert. Es zeigen:
- Figur 1: ein Blockschaltbild des Endoskops mit Steuergerät,

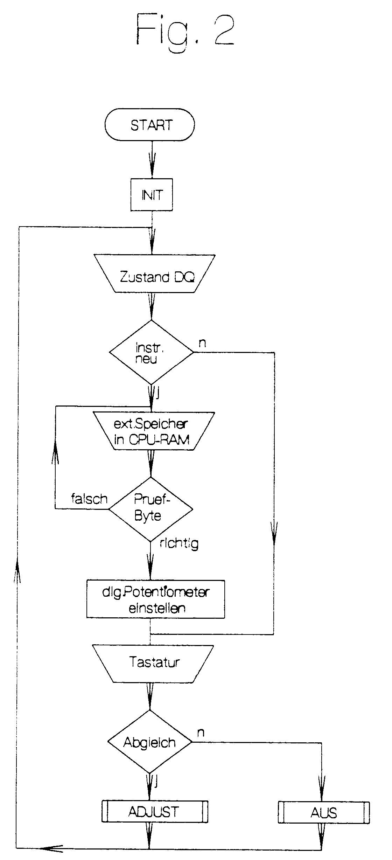

- Figur 2: ein Flußdiagramm der Instrumentenerkennung,
- Figur 3: ein Flußdiagramm der Abgleichparametereinstellung und
- Figur 4: ein Flußdiagramm der Betriebsparameterinstellung.

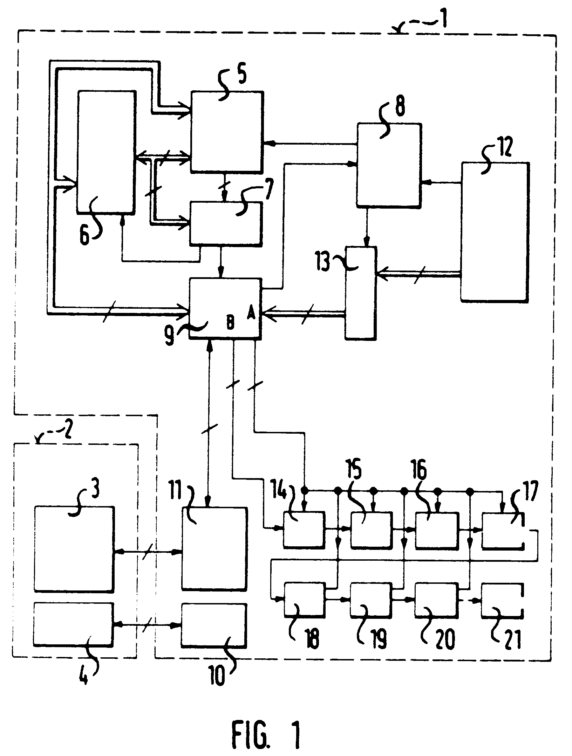

Das in Figur 1 dargestellte Endoskopsystem bestehend aus einem Videoendoskop 2 und einem Steuergerät 1 weist einen internen und einen externen Schaltungsteil auf. Dabei ist der letztere dem Instrument 2, d.h. dem Videoendoskop oder auch einem Kamerakopf zugeordnet, während der interne Schaltungsteil das Steuergerät 1 für die Steuerung des Instrumentes 2 und die Verarbeitung der von diesem kommenden Signale darstellt. Der dem Instrument 2 zugeordnete Schaltungsteil umfaßt einen CCD-Sensor 4 sowie einen durch ein ECONORAM gebildeten Schreib-Lesespeicher 3 (SRAM).

Das Steuergerät 1 weist eine zentrale Recheneinheit 5 (CPU) mit einem Speicher 6 (RAM) auf, der ein Dekoder 7, eine Steuerlogik 8 sowie eine Schnittstelle 9 (PIO) als Peripheriebausteine zugeordnet sind. Weiter umfaßt das Steuergerät 1 einen Videoprozessor 10 und ein Speicher-Interface 11, die mit dem Instrument 2 verbindbar sind, einen Eingabe- und Bedienteil bestehend aus einer Tastatur 12 und einem Register 13 sowie einer Reihe von digitalen Potentiometern 14 - 21.

Die Wirkungsweise der Gesamtschaltung ist folgende:
Wie aus dem Flußsdiagramm nach Figur 1 hervorgeht, erfolgt nach dem Start eine Initialisierung der an die CPU 5 angeschlossenen Peripheriebausteine 7 - 9. Danach wird der Zustand der Datenleitung, über die das SRAM 3 angeschlossen wird, überprüft. Ist kein Instrument angeschlossen, so liegt an der PIO 9 in Figur 1 low-Potential an. Wird nun ein Instrument angeschlossen, so wird die Datenleitung über den im Instrument befindlichen Widerstand auf high-Potential gelegt. Dieser Übergang von low- auf high-Potential tritt also nur auf, wenn ein Instrument angeschlossen wird. Im nächsten Programmschritt wird ausgewertet, ob dieser Übergang stattgefunden hat. Somit kann erkannt werden, ob ein Instrument 2 angeschlossen worden ist oder nicht. Falls ein Instrument angeschlossen worden ist, wird der externe Speicher 3 über den Port B der PIO 9 (Figur 1) eingelesen, und die aus dem SRAM 3 geladenen Daten an die digitalen Potentiometer 14 - 21 weitergegeben.

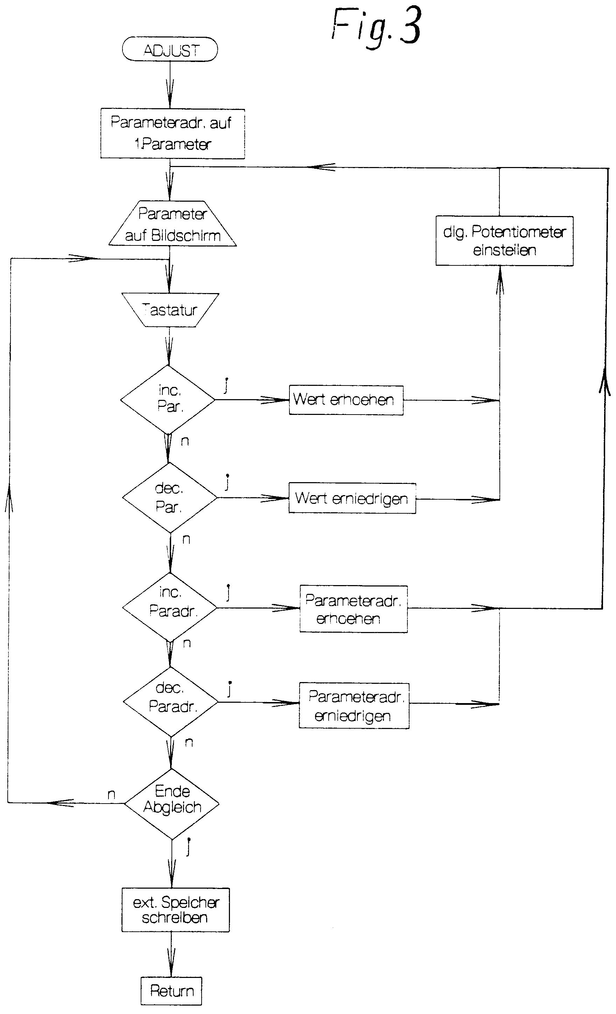

Im nächsten Programmschritt wird die Tastatur 12 eingelesen. Anhand der eingelesenen Daten wird entschieden, ob ein Abgleich durchgeführt werden soll. Soll kein Abgleich durchgeführt werden, so wird das eingelesene Zeichen als Text auf dem Bildschirm ausgegeben. Wird ein bestimmtes Zeichen eingegeben, so wird ein Abgleich im Unterprogramm "Adjust" (Figur 3) durchgeführt. Solange das Instrument 2 mit dem Steuergerät 1 verbunden ist, wird im nächsten Programmschritt die Tastatur 12 eingelesen. Der externe Speicher 3 wird erst wieder eingelesen, wenn das Instrument 2 vom Steuergerät 1 getrennt und dann erneut mit diesem verbunden wird.

In Figur 3 ist das Flußdiagramm für diesen Programmablauf dargestellt. Im ersten Programmschritt wird die Grundeinstellung, d.h. die Parameteradresse auf die Adresse des ersten abzugleichenden Parameters eingestellt. Danach wird der Wert des Parameters ausgegeben. Nach dem Einlesen der Tastatur 12 wird anhand der eingelesenen Zeichen entschieden ob der Wert des momentan adressierten Parameters oder die Parameteradresse erhöht oder erniedrigt wird, was einen Wechsel der entsprechenden Parameter zur Folge hat. Wird ein bestimmtes Zeichen eingegeben, so wird der Abgleich beendet und das externe SRAM 3 beschrieben. Wird ein Parameter verändert, so werden digitale Potentiometer 14 - 21 gesetzt, wodurch die Übernahme der veränderten Werte in das entsprechende digitale Potentiometer erfolgt.

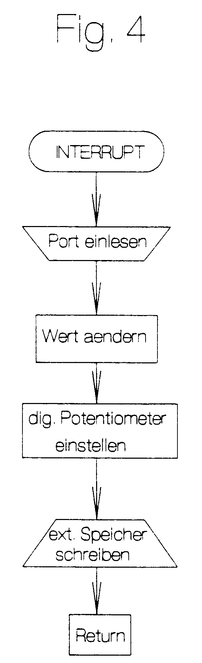

Die Betriebsparametereinstellung (Figur 4) erfolgt über ein Interrupt, der von der Steuerlogik 8, hervorgerufen durch einen STR-Impuls aufgrund einer Eingabe an der Tastatur 12 ausgelöst wird. Nach dem Auslösen des Interruptses unterbricht die CPU 5 das laufende Programm und springt in das Interrupt-Programm (Figur 4). Das Register 13 (Figur 1) hält den Tastenimpuls der jeweiligen Taste so lange fest, bis die Information durch die PIO 9 eingelesen wurde. Dieser Setz- bzw. Übernahmevorgang wird durch die Steuerlogik 8 koordiniert, die von der PIO 9 ein Signal (RDY) erhält, sobald der Einlesevorgang beendet ist.

Im ersten Programmschritt des Interruptprogramms wird der Port A der PIO 9 eingelesen. Im nächsten Programmschritt wird analysiert, welches Datenbit bei dem gelesenen Byte gesetzt ist. Der entsprechende Wert wird dann geändert und in den RAM 6 der CPU 5 geschrieben. Anschließend werden die digitalen Potentiometer 14 - 21 neu eingestellt und das externe SRAM 3 neu beschrieben. Demnach ist das Interrupt-Programm abgearbeitet und die CPU 5 arbeitet im laufenden Programm an der Stelle weiter, an der sie ins Interrupt-Programm gesprungen ist.

Die CPU 5 übernimmt die zeitliche Steuerung des Einstellvorganges und transferiert die Daten zwischen den übrigen Peripheriebausteinen. Der Dekoder 7 dekodiert die Adresse der an die CPU 5 direkt angeschlossenen Peripheriebausteine. Bei einem Schreib- oder Lesezyklus erkennt der Dekoder 7 anhand der Adress- und Steuerleitungen, welcher Peripheriebaustein angesprochen (selektiert) werden muß.

Im RAM 6 der CPU 5 werden die vom externen Speicher 3 eingelesenen Daten abgespeichert. Die PIO 9 übernimmt, gesteuert von der CPU 5, den Datentransfer zwischen dem Register 13, dem SRAM-Interface 11 und dem digitalen Potentiometern 14 - 21. Die Steuerlogik 8 sorgt dafür, daß die von Tasten 12 an dem Register 13 anstehenden Impulse zeitlich richtig im Register 13 gespeichert werden und das Register 13 nach Einlesen der Daten wieder zurückgesetzt wird.

Die digitalen Potentiometer 14 - 21 dienen zum Einstellen der vom Video-Prozessor 10 benötigten analogen Referenzspannungen. In den digitalen Potentiometern 14 - 21 erfolgt die Umwandlung des digitalen Parameterwertes in eine dem digitalen Parameterwert entsprechende Gleichspannung.

Das Interface 11 übernimmt die hardwaremäßige Anpassung zwischen PIO 9 und SRAM 3.

Der CCD-Sensor 4 liefert ein Videosignal, welches im Videoprozessor 10 zu einem zur Wiedergabe auf einem Monitor geeigneten Videosignal verarbeitet wird.

## Patentansprüche

1. Endoskop mit elektronischem Bildwandler, das über ein damit verbindbares Steuergerät an einen Monitor zur Bildwiedergabe anschließbar ist, mit einem dem Bildwandler endoskopseitig zugeordneten statischen Speicher, dadurch gekennzeichnet, daß der Speicher (3) ein Schreib- und Lesespeicher ist, daß der Speicher (3) zur Speicherung aller für das Steuergerät (1) erforderlichen Abgleichparameter des Bildwandlers (4) vorgesehen ist und daß der Speicher (3) mittels des Steuergeräts (1) belegbar und wieder abfragbar ist.

2. Endoskop nach Anspruch 1, dadurch gekennzeichnet, daß der Speicher (3) neben der Speicherung der Abgleichparameter auch zur Speicherung der am Steuergerät (1) einstellbaren Betriebsparameter und/oder weiterer Daten vorgesehen ist.

3. Endoskop nach einem der vorhergehenden Ansprüche, dadurch gekennzeichnet, daß der Speicher (3) zur Speicherung von Hersteller- und/oder Servicedaten vorgesehen ist.

4. Endoskop nach einem der vorhergehenden Ansprüche, dadurch gekennzeichnet, daß als Speicher (3) ein EEPROM vorgesehen ist.

5. Endoskop nach einem der vorhergehenden Ansprüche, dadurch gekennzeichnet, daß als Speicher (3) ein ECONORAM vom Typ DALLAS DS222X eingesetzt ist.

6. Steuergerät zum Verbinden eines Endoskops nach einem der vorhergehenden Ansprüche mit einem Monitor, dadurch gekennzeichnet, daß das Steuergerät (1) zur Abfrage des endoskopseitigen Speichers (3) ausgebildet ist und daß entsprechend den abgefragten Speicherdaten eine steuergeräteseitige Abgleichung an den Bildwandler (4) erfolgt.

7. Steuergerät nach Anspruch 6, dadurch gekennzeichnet, daß das Steuergerät (1) zur Übertragung von am Steuergerät (1) einstellbaren Betriebsparametern an und Eingabe in den endoskopseitigen Speicher (3) sowie zur Abfrage dieser Daten ausgebildet ist und daß die Betriebsparameter nach erfolgter Abfrage vom Steuergerät (1) übernehmbar sind.
